# FASCICULE DE BREVET EUROPEEN

(11) **EP 3 723 742 B1**
(45) Date de publication et mention de la délivrance du brevet: **30.08.2023**
(21) Numéro de dépôt: 18815771.3
(22) Date de dépôt: 17.12.2018
(51) Int. Cl.: A61K 31/13, A61K 31/14, A61P 25/22, A61K 45/06, A61K 31/138, A61K 31/135

(54) **UTILISATION DE LA FLUOROETHYLNORMEMANTINE POUR LA PREVENTION ET LE TRAITEMENT DE L'ANXIETE**
VERWENDUNG VON FLUOROETHYLNORMEMANTIN ZUR VORBEUGUNG UND BEHANDLUNG VON ANGSTZUSTÄNDEN
USE OF FLUOROETHYLNORMEMANTINE FOR THE PREVENTION AND TREATMENT OF ANXIETY

(30) Priorité: 15.12.2017 FR 1762290
(43) Date de publication de la demande: 21.10.2020
(73) Titulaire: REST THERAPEUTICS, 34095 Montpellier cedex 5 (FR)
(72) Inventeur: RUBINSTENN, Gilles, 75005 PARIS (FR)
(74) Mandataire: A.P.I. Conseil
(86) Numéro de dépôt international: PCT/EP2018/085333
(87) Numéro de publication internationale: WO 2019/115833

(56) Documents cités:
- WO-A1-2014/191424
- US-A1- 2014 057 988

## Description

La présente invention est relative à la découverte de l'utilisation d'un composant pharmaceutique dans le traitement de troubles liés à l'anxiété.

### Description de l'art antérieur :

De nombreuses molécules ont été développées pour le traitement de l'anxiété associée ou non à la dépression. Cependant ces molécules présentent de nombreux défauts, en particulier des effets secondaires importants, soit induisant une somnolence plus ou moins accrue, soit même suscitant des états psychotiques de type schizophrénique, soit demandant un temps significatif (plusieurs jours voire plusieurs semaines) de traitement avant l'apparition de l'effet thérapeutique avec un risque d'effet anxiogène pendant la période de mise en place du traitement.

### Anxiété

L'anxiété chez l'homme se traduit par un sentiment d'inquiétude, d'insécurité, de trouble physique ou psychique ou d'attente d'un danger indéterminé. Deux types d'anxiété sont aussi distingués : l'anxiété d'état et l'anxiété de trait. L'anxiété d'état est une anxiété « normale » liée à une situation ponctuelle dans laquelle se trouve le sujet. L'anxiété de trait est une anxiété « pathologique », constitutive d'un sujet. Elle est permanente et indépendante d'une situation particulière.

### Dépression

La dépression se traduit par des épisodes dits dépressifs au cours desquels le patient présente un abaissement de l'humeur, une réduction de l'énergie et une diminution de l'activité. Il existe aussi pendant une dépression, une altération de la capacité à éprouver du plaisir, une perte d'intérêt ainsi qu'une diminution rapide de la capacité à se concentrer. On observe également une fatigue importante même après un effort minime, des troubles du sommeil... Les patients ressentent fréquemment une diminution de l'estime de soi, des sentiments de culpabilité et de dévalorisation qui peuvent aller jusqu'à un désir de mort et l'élaboration de pensées suicidaires. Suivant le nombre et la gravité des symptômes, on définit trois degrés de sévérité de la dépression : dépression légère, moyenne et sévère.

Plusieurs troubles mentaux sont répertoriés dans la classification des maladies (CMI10) proposée par l'Organisation Mondiale de la Santé (OMS) qui associent anxiété et dépression :

### Les Troubles anxieux phobiques : F40

Le sujet présente des symptômes anxieux qui sont déclenchés exclusivement ou essentiellement par une ou des situations bien précises et sans dangerosité. De ce fait, le patient va chercher à éviter ces situations ou les endurer avec appréhension. Cette anxiété phobique est souvent associée à une dépression.

### Le Trouble anxieux et dépressif mixte :F41.2

Dans ce cas, le sujet présente à la fois des symptômes anxieux et des symptômes dépressifs sans prédominance nette de l'un ou de l'autre et sans que l'intensité de l'un ou de l'autre justifie un diagnostic séparé.

### Le Trouble obsessionnel compulsif (TOC) :F42

Ce trouble est caractérisé par des idées obsédantes ou des comportements compulsifs récurrents. Les pensées obsessionnelles font irruption dans la conscience du sujet de façon répétitive et stéréotypée, même lorsque celui cherche à les éviter. Les comportements compulsifs sont aussi des activités stéréotypées répétitives pour lesquelles le sujet ne ressent aucun plaisir direct mais qu'il ne peut s'empêcher d'accomplir. Ils ont pour but d'empêcher la survenue d'un évènement, objectivement peu probable, impliquant un malheur pour le sujet. Ce dernier reconnait généralement l'absurdité de son comportement et fait des efforts répétés pour le faire cesser. Ce trouble s'accompagne presque toujours d'une anxiété et d'une dépression.

### Etat de stress post-traumatique (ESPT) : F43.1

L'état de stress post-traumatique se développe à la suite de l'exposition (directe ou indirecte) du sujet à un événement traumatique dans un contexte de mort, de menaces de mort, de blessures graves ou d'agression sexuelle... Par définition menaçant et entraînant une réaction de peur intense, cet événement dit traumatique représente une situation de stress extrême. Il se traduit par un ensemble de symptômes et de comportements spécifiques. Par exemple : des reviviscences (souvenirs répétitifs et envahissants de l'événement, cauchemars...), des comportements d'évitement (évitement des souvenirs, des pensées, des personnes, des situations ... rappelant l'évènement traumatique), des altérations cognitives et émotionnelles (tendance à se blâmer, émotions négatives persistantes...) et enfin l'hyperactivation du système nerveux (hypervigilance, difficulté de sommeil...). Les personnes qui souffrent de ce syndrome développent aussi souvent une dépression profonde ou des idées suicidaires (Shalev, Am. J. Psychiatry 155,5, p 630-637, May 1998). Ce phénomène d'état de stress post-traumatique est lié à une distorsion profonde de l'encodage mémoriel des événements. Le souvenir est mémorisé à long terme mais de façon biaisée, avec une amnésie de certains aspects et une hypermnésie d'autres détails qui harcèlent le sujet.

Il existe donc un réel besoin de solutions nouvelles permettant d'adresser efficacement les troubles anxieux, couplés ou non à une dépression.

### Traitement des troubles associant l'anxiété et la dépression :

Les traitements conventionnels de l'anxiété, associée ou non à la dépression, incluent la thérapie comportementale, le changement de mode de vie mais aussi une thérapie pharmaceutique. Cependant un grand nombre des médicaments utilisés pour traiter ces désordres sont connus pour avoir soit des effets secondaires gênants, soit sont responsables d'une accoutumance ou d'une dépendance (Thèse A. Dubois 2015 Université de Bordeaux). On retrouve la majorité des solutions thérapeutiques employées actuellement pour traiter les désordres liés à l'anxiété, ainsi que certains de leur effets secondaires, dans la revue suivante: Guidelines for the pharmacological treatment of anxiety disorders, obsessive - compulsive disorder and posttraumatic stress disorder in primary care, B. Bandelow, International Journal of Psychiatry in Clinical Practice, 2012; 16: 77-84.

Parmi les toutes premières substances utilisées, on peut par exemple citer les dérivés du barbital, appelés barbituriques, qui étaient largement utilisés dans les années 1950 mais présentaient de fort risques de dépendance ou d'intolérance.

D'autres médicaments pour soigner l'anxiété et la dépression furent ensuite développés et notamment les benzodiazépines dont le chef de fil est le diazepam (Valium^{®}...). Ces molécules présentées initialement comme efficaces et d'une relativement bonne tolérance ont été et sont toujours beaucoup prescrites, notamment en France et aux Etats-Unis. Les benzodiazépines ont le pouvoir de se fixer sur un récepteur cellulaire, normalement réservé au GABA (acide γ-aminobutyrique). Ce composé chimique naturel est le principal inhibiteur du système nerveux central. En se fixant sur le récepteur cellulaire, les benzodiazépines augmentent l'affinité de ces récepteurs pour le GABA, et donc décuplent son pouvoir d'action. Cependant, il a été mis en évidence une importante pharmacodépendance à ces molécules ainsi que d'autres effets indésirables (altération des fonctions cognitives, apparition de troubles de nature schizophrénique, somnolence, syndrome de sevrage et phénomène de rebond...) et des solutions de remplacement ont donc été recherchées. Par ailleurs les benzodiazépines n'ont pas été trouvées efficaces dans le traitement des TOC et d'autres troubles associant anxiété et dépression.

Une autre classe de molécules anti-anxiété et anti-dépression a ensuite été développée fondée sur l'action d'un autre neurotransmetteur : la sérotonine (5-hydroxytryptamine ou 5-HT). C'est un neurotransmetteur libéré au moment de l'échange d'information entre un neurone émetteur et un neurone récepteur. Les ISRS sont des Inhibiteurs Sélectifs de la Recapture de la Sérotonine, on peut citer par exemple la fluoxetine (Prozac^{®}) ou la vortioxetine (Trintellix^{®}, Brintellix^{®}). Une partie de l'information entre neurone émetteur et récepteur se perd car le neurotransmetteur est re-capturé par le neurone émetteur et non disponible pour le neurone récepteur. Les ISRS agissent en empêchant la recapture de la sérotonine par le neurone transmetteur, la probabilité que la sérotonine soit reconnue par le neurone récepteur augmente, majorant ainsi la stimulation de ce neurone. Pour exploiter cette propriété, de nombreuses molécules ISRS ont été développées (voir par exemple le brevet EP0216555A2). Les résultats contre l'anxiété et la dépression ont été très prometteurs, mais là encore des effets secondaires ont été constatés (en particulier la levée des inhibitions comportementales...). Par ailleurs des preuves de l'efficacité des ISRS pour réduire la sévérité des symptômes de l'ESPT ont été apportées tout particulièrement dans le cas de la paroxétine et de la sertraline (P Carlier, Annales médico-psychologiques 166, 2008, 747-754). Cependant là encore des effets secondaires sont apparus (paranoïa, syndrome sérotoninergique...) ainsi qu'un certain nombre d'intolérances ou d'inefficacité justifiant la nécessité d'associer les ISRS avec d'autres antidépresseurs (stabilisateur de l'humeur...).

Un autre neurotransmetteur, le glutamate, fait aussi l'objet de recherches intensives pour développer de nouveaux médicaments. Il est aujourd'hui admis que le glutamate active plusieurs types de récepteurs (Mémoire EPHP de M. Teigell-Webb) : le glutamate agit sur trois récepteurs ionotropes, caractérisés et dénommés par le nom de leur agoniste le plus sélectif: les récepteurs N-méthyl-D-aspartate (NMDA), kainate (KA), et a-amino-3-hydroxy-5-métyl-4-isoxazolepropionate (AMPA). L'étude du récepteur NMDA s'est développée plus rapidement car les recherches ont montré que les récepteurs NMDA étaient impliqués dans d'intéressants aspects des fonctions cérébrales. Les récepteurs NMDA jouent des rôles physiologiques très variés : différentiation neuronale, formation des connections synaptiques au cours du développement, plasticité synaptique chez l'adulte. Il a en particulier était montré chez l'adulte que le récepteur NMDA est impliqué dans l'apprentissage et la mémoire à court terme; il participe dans certaines régions de l'hippocampe au renforcement de l'efficacité synaptique (ou potentialisation à long terme) qui servirait de support au stockage de l'information. Enfin l'implication du récepteur NMDA dans l'augmentation des comportement anxieux suite à l'exposition à un stress (R.E. Adamek et al, Physiology & Behavior, Vol. 65, Nos. 4/5, pp. 723-737, 1999) en fait une cible de choix pour le développement de traitement pour l'ESPT. Notons que l'originalité du récepteur glutamate est que, contrairement à d'autres récepteurs-canaux activés par un ligand, on observe la dépendance duale de la liaison d'un agoniste (et d'un co-agoniste) et du potentiel de membrane pour ce récepteur. Sur le site du glutamate, se lient les agonistes et les antagonistes compétitifs. Les antagonistes non-compétitifs des récepteurs NMDA sont des molécules capables de se lier sur un site de régulation différent de celui du glutamate. Ainsi, dans le cas du traitement de l'anxiété, ont été développés par exemple des peptides inhibiteurs du récepteur NMDA (Brevet EP2175873).

Par ailleurs, les composés dits "bloqueurs de canal" sont des agents qui se lient à l'intérieur du canal associé au récepteur NMDA lorsque ce dernier est en position "ouvert" (cellule dépolarisée et glutamate lié). Parmi les « bloqueurs de canaux » les plus connus, se trouvent la kétamine, la memantine... Cette dernière molécule a déjà montré des effets neuroprotecteurs. Elle est par exemple déjà utilisée pour le traitement de la maladie d'Alzheimer, mais elle a aussi été testée pour traiter les désordres comportementaux des enfants (autisme) comme par exemple dans le brevet US2010081723A1.

Notons aussi que les antagonistes non-compétitifs se liant sur le site de la phencyclidine (PCP) du récepteur NMDA comme par exemple la kétamine, la dizocilpine, la memantine ont aussi été testés pour soulager l'anxiété liée à l'état de test post-traumatique (Arthur L. Womble, AANA Journal April 2013 Vol. 81, No. 2.). En ce qui concerne la kétamine et la dizocilpine, les résultats positifs de ces études sont fortement assombris par les effets secondaires (hallucinations, flashbacks, paranoïa...). Notons que la kétamine inhibe fortement le filtrage sensoriel.

Le développement de la kétamine est néanmoins poursuivi par plusieurs équipes qui ont mis en évidence un effet prophylactique intéressant quand la molécule est injectée en une seule fois, 1 semaine avant le conditionnement à la peur chez le rongeur (C. Denny et al, in Neuropsychopharmacology (2017), 1-13), se traduisant par une réduction significative de la tétanie, durant la phase de stabilisation. Néanmoins, l'effet n'est visible qu'à des doses élevées pour ce modèle (30mg/kg) et ne sont pas reproduites si le traitement est concomitant (ou précède juste) le conditionnement ou si le traitement protecteur est fait trop tôt (1 mois à l'avance).

US 2014/057988 A1 propose également l'utilisation de la kétamine comme antagoniste au récepteur NMDA pour l'inhibition, l'amélioration ou le traitement du développement de l'anxiété légère, l'anxiété légère étant définie dans ce document comme un sentiment de malaise ou d'appréhension. Ce document évoque et cite l'utilisation de la mémantine dans le traitement de la démence, or la mémantine n'a pas d'effet anxiolytique et présente des effets secondaires indésirables de type schizophréniant.

WO 2014/191424 décrit l'utilisation de dérivés de la mémantine, notamment du 2-fluoroethyl normémantine, dans le traitement de maladies neurodégénératives telles que l'Alzheimer ou la maladie de Parkinson.

La memantine a elle aussi été utilisée pour traiter les troubles de l'anxiété, associés ou non à une dépression comme l'ESPT et les troubles obsessionnels compulsifs (G. Sani, review article CNS Drugs 2012; 26 (8): 663-690). De manière générale, l'action de la memantine est positive lorsqu'elle est utilisée en association (thérapie d'augmentation) avec d'autres molécules. Son efficacité est beaucoup plus faible et controversée lorsqu'elle est utilisée en monothérapie. Par exemple pour traiter les TOC, la memantine a été associée à des ISRS comme la fluoxétine ou le citalopram, ou bien avec d'autres antidépresseurs de type tricyclique comme la clomipramine. Généralement l'utilisation de memantine comme traitement complémentaire est bien tolérée pour de faibles doses journalières (inférieures à 15 mg/jour environ). Mais des effets secondaires ont été observés comme un état dysphorique, de la somnolence, des nausées lorsque des doses trop importantes étaient administrées. Dans le cas des traitements de l'ESPT, les résultats d'utilisation sur les modèles animaux de l'ESPT n'ont pas donné de résultats convaincants, mais pour quelques études de cas d'ESPT humain, la memantine utilisée comme traitement additif a semblé améliorer les symptômes de ces patients. En tout état de cause la mémantine utilisée seule n'est pas capable de réduire ou traiter l'anxiété, quelle soit légère ou sévère, qu'elle soit ou non associée à une dépression. Parmi, les utilisations en association avec d'autres molécules, notons aussi l'utilisation de la memantine associée à des inhibiteurs de la glycoprotéine de la vésicule synaptique SV2A (brevet US 2016030391A1) ainsi que la combinaison memantine/mélatonine (brevet EP 2905021 A1).

Par ailleurs, même si elle semble présenter peu d'effets secondaires et être bien tolérée, la memantine semble avoir des effets hyper-tranquillisants non recherchés (somnolence, fatigue, apathie...), et entraîner aussi à haute dose une augmentation des étourdissements (NR Swerdlow, Neuropsychopharma., 2009, 34, 1854-1864) ainsi qu'un effet rebond maintes fois signalé par des patients sur les forums internet. En outre, il est bien connu que la mémantine, en particulier à hautes doses, ou le fait qu'elle n'est pas anxiolitique, est capable d'induire des effets secondaires de type schizophrénie.

Enfin, dans le cas des récepteurs NMDA, il apparaît que les différents antagonistes n'agissent pas d'une seule façon, de sorte que les intérêts et les désavantages d'un antagoniste ne se retrouvent pas nécessairement pour un autre antagoniste, et ce même si les structures sont proches.

Pour le traitement secondaire des troubles de l'ESTP, une dernière classe de molécules a été proposée : les agents catécholaminergiques (F. Ducrocq, L'Encéphale, 2005 ; 31 : 212-26). Ils ont été mis en oeuvre dans des interventions pharmacologiques visant à supprimer directement l'hyperréactivité noradrénergique, et étudiés au travers d'essais ouverts et en double aveugle sur de petits effectifs. Ces agents sont en particulier la clonidine, la guanfacine, la prazozine et le propranolol. Parmi ces molécules, le propranolol a fait l'objet à ce jour des travaux les plus déterminants. Ce B-bloqueur adrénergique non sélectif a ainsi été théoriquement considéré dès 1994 comme capable de réduire les phénomènes de consolidation de la mémoire émotionnelle dans les suites immédiates de la confrontation à un événement traumatique (Cahill L., Nature 1994 ; 371 : 702-4.). Quelques années auparavant, Famularo et ses collègues avaient testé la molécule dans le trouble constitué chez l'enfant en construisant une étude basée sur un design B-A-B (off-on-off) de 3 phases de 4 semaines de traitement entrecoupées de phases de 2 semaines de sevrage. Dans cette étude, 8 des 11 enfants enrôlés ont présenté une amélioration significative de leurs symptômes psychotraumatiques (répétition et hyperréactivité neurovégétative) lors des phases de traitement, mais cette amélioration disparaît lors du sevrage (Famularo R, Am J Dis Child 1990 ; 142 : 1244-7.). Ce dernier élément limite considérablement l'intérêt du propranolol au-delà de l'accompagnement de thérapies cognitives de l'ESPT.

L'absence de solution efficiente pour le traitement de l'ESPT est également démontrée par la situation des prescriptions. Une étude récente (Tonix Pharmaceuticals Holding Corp. (TNXP) FORM 8-K | Current report Dec 8 2016. UNITED STATES SECURITIES AND EXCHANGE COMMISSION Washington, D.C. 20549 Pursuant to Section 13 or 15(d) of the Securities Exchange Act of 1934) a ainsi montré qu'aux Etats-Unis, alors que seule la classe des ISRS est autorisée par la FDA pour le traitement de l'ESTD, la première classe de médicaments prescrits est la classe des benzodiazépines pour 55% des patients, contre 53% pour les ISRS.

Face au besoin de solution efficientes du traitement de l'ESPT, la demanderesse a donc imaginé d'utiliser une molécule dérivée structurellement de la memantine mais présentant un moment dipolaire très différent généré par l'introduction d'un atome de fluor pour traiter l'anxiété, associée ou non à la dépression. Dans le cas spécifique de l'ESPT, la demanderesse a découvert de façon surprenante que la molécule pouvait agir aussi bien en protection, c'est-à-dire par une administration concomitante à l'évènement traumatique, que pour un traitement ultérieur voire retardé. La demanderesse a en outre déterminé que la molécule ne présentait pas les nombreux effets secondaires non souhaités des traitements de l'état de l'art ou proposés en cours d'investigation.

C'est ainsi un premier objet de l'invention que de proposer un composé de formule (I) ou un de ses sels pharmaceutiquement acceptable 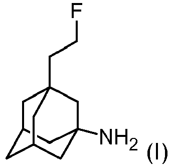 pour son utilisation pour le traitement ou la prévention d'un trouble anxieux chez un patient.

Le composé (I) pour son utilisation selon l'invention est caractérisé en ce qu'il est dénué d'effet d'induction schizophrénique.

Par l'expression « dénué d'effet d'induction schizophrénique » il est entendu que le composé (I) n'induit pas - aux doses permettant le traitement ou la prévention d'un trouble anxieux - de schizophrénie ou de symptômes associés tels que délires schizophréniques, délires paranoïdes, hallucinations, troubles de l'attention, repli sur soi, incohérence ou encore désorganisation.

Dans un second mode de réalisation, l'invention vise un composé pour son utilisation selon le premier mode de réalisation, caractérisé en ce que le sel pharmaceutiquement acceptable correspond à la formule (II) 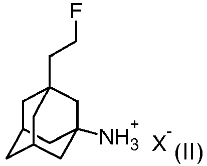 dans laquelle, X⁻ désigne un contre anion choisi dans le groupe constitué par les ions chlorure, bromure, iodure, acétate, méthane sulfonate, benzene sulfonate, camphosulphonate, tartrate, dibenzoate, ascorbate, fumarate, citrate, phosphate, salicylate, oxalate, bromohydrate et tosylate.

C'est aussi un troisième objet de l'invention que de proposer un composé pour son utilisation selon l'un des modes de réalisations 1 ou 2, caractérisé en ce que le trouble anxieux est associé à une dépression.

Le composé est caractérisé en ce qu'il est dénué d'effet d'induction schizophrénique.

C'est l'objet d'un quatrième mode de réalisation de l'invention que de fournir un composé pour son utilisation selon l'un des modes de réalisations 1 ou 2, caractérisé en ce que le trouble anxieux observé est associé à des insomnies.

L'invention vise aussi dans un cinquième mode de réalisation, un composé pour son utilisation selon l'un des modes de réalisations 1 à 4, caractérisé en ce que le trouble anxieux est choisi dans le groupe constitué par l'état de stress post-traumatique (ESPT), la phobie spécifique, la phobie sociale, le trouble d'anxiété généralisée, le trouble panique avec ou sans agoraphobie et le trouble obsessionnel compulsif.

Le composé est caractérisé en ce qu'il est dénué d'effet d'induction schizophrénique.

Dans un sixième mode de réalisation, l'invention vise encore un composé pour son utilisation selon l'un des modes de réalisations 1 à 5, caractérisé en ce qu'il est utilisé pour un traitement de prévention de l'apparition des troubles anxieux liés à un stress post traumatique.

Le composé est caractérisé en ce qu'il est dénué d'effet d'induction schizophrénique.

Le traitement des troubles anxieux liés à un stress post-traumatique comprend en particulier le traitement de toute combinaison d'au moins un des critères du DSM 5 sur le PTSD choisi dans le groupe consistant en : la reviviscence, l'évitement, les cognitions et l'humeur négatives et l'hyperréactivité.

La reviviscence fait particulièrement référence aux cauchemars associés. Les cognition et l'humeur négatives font référence aux états dépressifs.

Selon les modes de réalisation de l'invention, le composé (I) ou (II) peut être administré sous forme de doses comprenant 1 mg à 1000 mg de composé (I) ou (II), par exemple 5 à 250 mg, par exemple entre 5 à 100 mg et, de préférence de 5 à 30 mg de composé (I) ou (II). Les doses en questions peuvent être administrées par exemple de 1 à 4 fois par jour, par exemple 1 fois, par exemple 2 fois, en particulier 3 fois, voire encore 4 fois.

Selon un mode de réalisation particulier, le composé (I) pour son utilisation selon l'invention est caractérisé en ce qu'il est dénué d'effet d'induction schizophrénique. Dans un septième mode de réalisation, l'invention vise un composé pour son utilisation selon le mode de réalisation 6, caractérisé en ce que le traitement comprend l'administration du composé de manière concomitante à l'évènement traumatique causant le stress post traumatique.

Un huitième mode de réalisation de l'invention vise aussi un composé pour son utilisation selon le mode de réalisation 6, caractérisé en ce que le traitement comprend l'administration du composé pendant les 4 à 18 semaines suivant l'évènement traumatique causant le stress post traumatique.

Le composé pourra être administré pendant 4, 5, 6, 7, 8, 9, 10, 11, 12, 13, 14, 15, 16, 17 ou 18 semaines.

C'est un neuvième mode de réalisation que de fournir un composé pour son utilisation selon l'un des modes de réalisation 1 à 5, caractérisé en ce que le trouble anxieux est lié à un stress post traumatique, en particulier un stress post traumatique installé.

Le composé est caractérisé en ce qu'il est dénué d'effet d'induction schizophrénique.

Dans un dixième mode de réalisation, l'invention vise un composé pour son utilisation selon le mode de réalisation 9, caractérisé en ce que le patient présentant un trouble anxieux suit une thérapie antidépressive choisie parmi les thérapies comportementales et les traitements pharmacologiques antidépresseurs.

Par traitement pharmacologique antidépresseur il est entendu l'administration de d'au moins une molécule chimique connue pour le traitement de la dépression. Parmi celles-ci on peut citer les inhibiteurs de recapture de la sérotonine tels que la fluoxetine, la vortioxetine, la fluvoxetine, la sertraline, la paroxetine, le citalopram, l'escitalopram, la duloxetine, le milnacipram, la venlafaxine, l'indalpine, la zimelidine, la dapoxetine.

Dans un onzième mode de réalisation, l'invention vise un composé pour son utilisation selon le mode de réalisation 10, caractérisé en ce que le patient suit un traitement à base de propanolol.

Selon un douzième mode de réalisation, l'invention vise un composé pour son utilisation selon l'un des modes de réalisation 1 à 11, caractérisé en ce que le trouble anxieux est accompagné de crises et d'épisodes d'angoisse aigus.

L'invention vise aussi, dans un treizième mode de réalisation, un produit de combinaison, en particulier pour une utilisation simultanée, séparée ou étalée dans le temps, comprenant les composants suivants : un composant (A) comprenant un composé de formule (I) ou un de ses sels pharmaceutiquement acceptable et au moins un excipient pharmaceutiquement acceptable 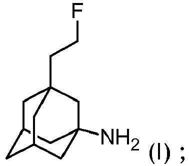 un composant (B) comprenant du propranolol et au moins un excipient pharmaceutiquement acceptable.

Selon un mode particulier du produit selon l'invention, il est caractérisé en ce qu'il est dénué d'effet d'induction schizophrénique.

C'est un quatorzième mode de réalisation de l'invention que de fournir un produit de combinaison comprenant les composants suivants : un composant (A) comprenant un composé de formule (I) ou un de ses sels pharmaceutiquement acceptable 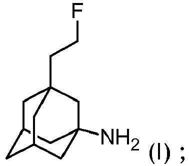 un composant (C) comprenant un antidépresseur en particulier un des inhibiteurs de recapture de la sérotonine décrit dans le mode de réalisation 10; dans lequel chacun des composants (A) et (C) est formulé avec au moins un excipient pharmaceutiquement acceptable.

Selon un mode particulier du produit selon l'invention, il est caractérisé en ce qu'il est dénué d'effet d'induction schizophrénique.

Dans un quinzième mode de réalisation, l'invention vise aussi un produit selon le mode de réalisation 13, comprenant en outre en combinaison, un composant (D) comprenant de la ketamine formulée avec un excipient pharmaceutiquement acceptable.

Dans un seizième mode de réalisation, l'invention vise aussi un produit selon le mode de réalisation 14, comprenant en outre en combinaison, un composant (D) comprenant de la kétamine formulée avec un excipient pharmaceutiquement acceptable.

Dans un dix-septième mode de réalisation, l'invention fournit aussi un produit selon le mode de réalisation 13, dans lequel les composants (A) et (B) sont adaptés pour une administration simultanée, séparée ou étalée dans le temps.

Dans un dix-huitième mode de réalisation, l'invention vise un produit selon le mode de réalisation 14, dans lequel les composants (A) et (C) sont adaptés pour une administration simultanée, séparée ou étalée dans le temps.

Dans un dix-neuvième mode de réalisation, l'invention vise un produit selon le mode de réalisation 15, dans lequel les composants (A), (B) et (D) sont adaptés pour une administration simultanée, séparée ou étalée dans le temps.

Selon un vingtième mode de réalisation, l'invention vise un produit selon le mode de réalisation 16, dans lequel les composants (A), (C) et (D) sont adaptés pour une administration simultanée, séparée ou étalée dans le temps.

Dans un vingt-et-unième mode de réalisation, l'invention concerne un produit selon l'un des modes de réalisation 13 à 20, pour son utilisation dans la prévention ou le traitement d'un trouble anxieux chez un patient.

Selon un vingt-deuxième mode de réalisation, l'invention concerne un produit pour son utilisation selon le mode de réalisation 21, caractérisé en ce que le patient suit une thérapie cognitive comportementale.

Selon un vingt-troisième mode de réalisation, l'invention vise encore un produit pour son utilisation selon le mode de réalisation 22, caractérisé en ce que le traitement du trouble anxieux est postérieur à la thérapie cognitive comportementale.

Dans un vingt-quatrième mode de réalisation, l'invention vise un produit selon l'un des modes de réalisation 13 ou 17, pour son utilisation dans la prévention ou le traitement d'un trouble anxieux chez un patient, ledit patient ayant subi des abus répétés, en particulier durant l'enfance.

Selon un vingt-cinquième mode de réalisation, l'invention fournit un produit pour son utilisation selon le mode de réalisation 24, caractérisé en ce que ledit patient suit une thérapie cognitive comportementale.

Selon un vingt-sixième mode de réalisation, l'invention vise encore un produit pour son utilisation selon le mode de réalisation 25, caractérisé en ce que les composants (A) et (B) sont utilisés de manière séparés dans le temps et postérieurement à la thérapie cognitive comportementale.

En effet, la demanderesse a découvert récemment un dérivé polarisé de la memantine, la 2-fluoroéthylnormemantine (FENM) (cf formule I) qui possède une affinité améliorée pour les récepteurs NMDA (brevet WO2014191424A1).

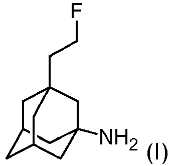

La molécule (I) peut-être en équilibre avec une forme protonée de formule (II), dans laquelle X⁻ désigne un contre anion issu du milieu biologique ou choisi parmi les ions chlorure, bromure, iodure, acétate, méthane sulfonate, benzene sulfonate, camphosulphonate, tartrate, dibenzoate, ascorbate, fumarate, citrate, phosphate, salicylate, oxalate, bromohydrate, tosylate. Le produit de formule (II) est donc un sel pharmaceutiquement acceptable du produit de formule (I).

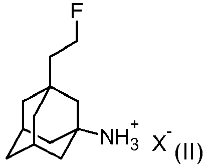

On notera par exemple un sel pharmaceutique acceptable particulièrement préféré de la fluoroéthylnormemantine dont le contre anion est un ion chlorure, i.e. FENM-HCl.

Les tests d'investigation conduits lors du développement de la FENM comme biomarqueur ont montré que cette molécule se lie de manière efficiente sur le site PCP (phencyclidine) du récepteur NMDA lorsque le canal de celui-ci est dans l'état « ouvert », ceci prévient en particulier un afflux excessif de Ca²⁺ dans la cellule. De façon intéressante, cette affinité améliorée n'est pas due à une liaison forte avec le récepteur NMDA, mais à la grande spécificité de la molécule pour les zones du cerveau où se situe le récepteur, ainsi qu'à son temps de résidence prolongé dans ces zones. De fait, la corrélation existante entre la FENM et les anticorps monoclonaux démontrant l'efficience du bio-marquage est perdue en cas d'injection de kétamine. Ces explorations préliminaires ont aussi montré un passage rapide de la barrière sang/cerveau.

De manière surprenante, la demanderesse a mis en évidence que le composé (I) (ou un de ses sels, particulièrement le FENM-HCl) peut être utilisé, pour le traitement de l'anxiété, associée ou non à la dépression. Dans le cas spécifique de l'ESPT, la demanderesse a découvert de façon surprenante que le composé (I) ou un de ses sels pharmaceutiques acceptable pouvait agir aussi bien en protection, c'est-à-dire par une administration concomitante à l'évènement traumatique, que lors d'un traitement ultérieur voire retardé. La demanderesse a en outre déterminé que la molécule ne présentait pas les nombreux effets secondaires non souhaités des traitements de l'état de l'art ou proposés en cours d'investigation.

La demanderesse a aussi mis en évidence une activité anxiolytique du produit (I) ici décrit (test du light dark box et gradient lumineux). A contrario, il a été démontré dans les mêmes conditions que la Memantine n'a aucune activité anxyolytique.

Le produit (I) ici décrit a une activité très significative anxiolytique, et son affinité avec le récepteur NMDA (mesure en Ki) étant du même ordre de grandeur que celle de la mémantine.

Dans le test de la boîte éclairée-obscure basé sur l'aversion innée des rongeurs pour la lumière, l'augmentation du temps passé dans le compartiment éclairé est considérée comme rendant compte d'un niveau d'anxiété plus faible ; ce qui est constaté pour les rongeurs traités avec le produit (I) selon l'invention. Aucun effet n'est constaté pour la mémantine pour laquelle il est donc conclu et confirmé qu'elle n'a aucun effet anxiolytique.

En outre, les inventeurs ont aussi démontré grâce à un test de filtrage sensoriel, que contrairement à la mémantine, le produit (I) n'induit aucun effet d'induction schizophrénique.

C'est ici une caractéristique clé du produit (I), à savoir de traiter l'anxiété, associée ou non à la dépression, tout en n'induisant pas d'effet d'induction schizophrénique.

Dans le cadre de la présente invention, l'expression « administration concomitante à l'évènement traumatique », on entend que le composé (I) est administré dans une fenêtre de temps allant de 48 heures avant l'évènement traumatique à 72 heures après l'évènement traumatique. Cette « administration concomitante à l'évènement traumatique » pourra aussi être comprise entre 48 heures avant et 48 heures après l'évènement traumatique, ou encore 48 heures avant et 24 heures après l'évènement traumatique, ou encore 24 heures avant et 24 heures après l'évènement traumatique, ou encore 24 heures avant et 48 heures après l'évènement traumatique ou encore 24 heures avant et 72 heures après l'évènement traumatique. Dans tous les cas, l'administration concomitante pourra être prolongée pendant 4, 5, 6, 7, 8, 9, 10, 11, 12, 13, 14, 15, 16, 17 ou 18 semaines pour aboutir à une protection plus efficiente.

La présente invention a donc pour premier objet de fournir une méthode pour traiter les patients souffrant de troubles de l'anxiété, associés ou non à une dépression, en administrant notamment un composé de formule (I) ou un sel pharmaceutiquement acceptable de formule (II). En particulier, la méthode n'induit pas d'effet d'induction schizophrénique.

La présente invention a aussi pour objet l'utilisation un composé de formule (I) ou un sel pharmaceutiquement acceptable de formule (II), dans la fabrication d'un médicament pour le traitement des troubles de l'anxiété, associés ou non à une dépression.

Comme décrit dans les exemples suivants, l'activité anxiolytique et possiblement antidépressive des composés de formule (I) peut être évaluée en utilisant les paradigmes animaux appropriés.

Ainsi, grâce au paradigme expérimental de la boîte éclairée-obscure, la demanderesse a découvert que les composés de formule (I) ou (II) selon la présente invention ont des propriétés anxiolytiques importantes et ce dès la première injection pour un dosage de 10mg/kg, contrairement à la memantine qui induit un temps de présence augmenté dans le compartiment obscure. Pour comparaison, une molécule comme la fluoxetine présente une telle activité anxiolytique pour des doses de 5mg/kg mais seulement au bout de 7 jours de traitement.

Cette découverte a été vérifiée, par la mise en oeuvre du test de l'espace ouvert en présence de gradient lumineux. Dans ces conditions, le composé selon l'invention limite significativement l'évitement de la zone la plus éclairée du champ par le rongeur lors de la phase d'exposition, voire l'annule complètement, en particulier à la dose 10mg/kg.

De même, grâce au test de la nage forcée, la demanderesse a découvert que le composé selon la présente invention induit une importante diminution du temps d'immobilité chez les rats comme lorsqu'ils sont traités avec la memantine ou la kétamine, ce qui traduit un comportement antidépresseur de ces molécules et composés.

La demanderesse a aussi confirmé cet intérêt surprenant d'un composé selon l'invention dans le traitement des troubles de l'anxiété, associés ou non à une dépression en les utilisant dans un test de conditionnement à la peur avec phase d'extinction et de stabilisation. Ce paradigme utilisé couramment pour tester l'efficacité des traitements de l'ESPT, a montré qu'un composé selon la présente invention, de manière surprenante, permet, en particulier aux doses de 10mg/kg et 20mg/kg de protéger le patient contre la rémanence des troubles lors de la phase de stabilisation, et ce plus particulièrement lorsque le composé est administré de façon concomitante à la phase de conditionnement. Dans le même test, aucun effet n'est observé ou pour le propranolol. Les effets observés pour la FENM sont équivalents ou supérieurs à ceux observé par C. Denny pour la ketamine injectée en quantité très supérieure, une semaine avant le conditionnement. Toujours de manière surprenante, la demanderesse a constaté qu'un effet bénéfique similaire en intensité, et plus particulièrement pour les mêmes doses, pouvait être observé quand le composé selon l'invention était administré avant la phase de stabilisation, soit lors d'un syndrome d'ESPT consolidé. Dans le même test, aucun effet n'est observé ou pour le propranolol.

Les composés selon l'invention sont, enfin, également efficients pour l'élimination des troubles rémanents, en particulier lorsqu'ils sont administrés lors de la phase d'extinction, plus particulièrement à la dose de 20mg/kg. Dans ce test, le propranolol diminue la tétanie pendant la phase d'extinction mais cet effet bénéfique est perdu lors de la phase de stabilisation.

Par ailleurs, on notera également qu'un effet rebond significatif est observé lors de la phase de stabilisation pour la memantine lorsqu'elle est administrée lors de la phase d'extinction. Cet effet, outre qu'il démontre des mécanismes d'action différents pour la memantine et la FENM, est en accord avec l'inefficacité observée en clinique de la memantine pour le traitement de l'ESPT.

Par ailleurs, la demanderesse a observé que lorsque les effets bénéfiques d'un composé selon l'invention étaient constatés, aucun effet d'inhibition de l'effet pré-impulsion n'était enregistré, tandis que la memantine produit un effet d'inhibition. Cette absence d'effet d'induction schizophrénique est très favorable au développement d'un nouveau traitement de l'anxiété, associée ou non à une dépression, dans la mesure où cet effet d'induction schizophrénique est, lui, observé pour la plupart des molécules proposées par ailleurs (dont les benzodiazépines en général et le diazepam en particulier, ainsi que la ketamine).

Le composé selon l'invention peut être administré de diverses manières afin d'obtenir les effets anxiolytiques ou antidépressifs. Le composé selon l'invention peut être administré seul ou sous la forme de préparations pharmaceutiques, au patient à traiter, soit de manière orale ou de manière parentérale (sous-cutanée ou par intraveineuse...).

La quantité du composé administrée peut varier et peut être n'importe quelle quantité responsable d'effets anxiolytiques ou/et antidépressifs. Suivant le patient et le mode d'administration, la quantité de composé administrée peut varier dans une large gamme entre 0.01 mg/kg jusqu'à 20 mg/kg, de préférence entre 0.05 mg/kg et 15 mg/kg du poids du patient par dose.

Les doses unitaires de composé selon l'invention peuvent contenir par exemple de 5 mg à 1000 mg, de préférence de 5 à 30 mg, et peuvent être administrées par exemple de 1 à 4 fois par jour.

La présente invention a pour objet l'utilisation d'un médicament formulé à partir du composé selon l'invention en association avec une méthode thérapeutique pharmaceutique ou cognitive comportementale pour le traitement des troubles de l'anxiété, associés ou non à une dépression.

Par « thérapie cognitive comportementale » il est entendu, au sens de la présente invention, les psychothérapies cognitives comportementales (ou thérapies cognitivo-comportementales, TCC) regroupant un ensemble de traitements des troubles psychiatriques (notamment addictions, psychoses, dépressions et troubles anxieux) qui partagent une approche selon laquelle la thérapeutique est basée sur les connaissances issues de la psychologie scientifique. Elle obéit à des protocoles relativement standardisés. Elle évalue souvent l'évolution du patient au cours de la thérapie. Elle accepte la démarche de médecine basée sur les faits. Les TCC ont pour particularité de s'attaquer aux difficultés du patient dans « l'ici et maintenant » par des exercices pratiques centrés sur les symptômes observables au travers du comportement et par l'accompagnement par le thérapeute qui vise à intervenir sur les processus mentaux dits aussi processus cognitifs, conscients ou non, considérés comme à l'origine des émotions et de leurs désordres. La standardisation de la pratique des TCC a contribué à la reconnaissance de leur efficacité par leur caractère reproductible qui est une des exigences de la démarche scientifique. Elles sont particulièrement indiquées pour les troubles anxieux (notamment les phobies) et les addictions.

En particulier, la demanderesse propose de mettre en oeuvre une combinaison de l'administration prophylactique de kétamine et d'un médicament contenant un composé selon l'invention postérieurement à l'exposition traumatique, afin de traiter l'ESPT. De même, la demanderesse propose de mettre en oeuvre une combinaison de l'administration de propranolol associée à une thérapie cognitive pendant une période de traitement initial de l'ESPT et d'un traitement par un médicament contenant le composé selon l'invention pendant la phase de sevrage, afin de traiter les troubles de l'anxiété présent chez les patients ayant subi des abus, en particulier durant l'enfance.

### EXEMPLES

### Modèles animaux :

La mise au point de médicaments pour traiter une maladie passe souvent par l'utilisation de modèles animaux permettant de tester dans un premier temps leur efficacité et leur toxicité avant de la tester chez l'homme.

Les modèles animaux sont réputés valides lorsqu'ils respectent les trois critères suivants : la validité de prédiction, l'isomorphisme des symptômes et la validité de construction. Bien entendu, les résultats issus de ces modèles animaux ne doivent pas dépendre des expérimentateurs et l'utilisation de moyens de conditionnement et d'observations automatisées est de plus en plus développée (utilisation de cellules photoélectriques, vidéotracking...).

### Utilisation des modèles animaux pour l'anxiété

Dans le domaine de l'anxiété, les modèles animaux sont utilisés depuis de très nombreuses années et améliorés au fur et à mesure du développement des nouvelles techniques d'observation ainsi que des biais observés dans leur validité (voir par exemple le cours de M Roy « Modèles animaux des pathologies anxieuses » disponible en ligne http://psychobiologierouen.free.fr/?page_id=40). La plupart des modèles animaux sont développés avec des rongeurs.

Dans le cas de l'anxiété, plusieurs modèles animaux permettent de la provoquer et de la mesurer en assujettissant ces derniers à des situations de danger soit par une simple exposition à la nouveauté, soit en les soumettant à une situation préalablement renforcée. Ces modèles animaux ont été validés notamment en vérifiant leur caractère prédictif de la réponse chez l'être humain.

Ainsi pour tester l'anxiété d'état, plusieurs modèles animaux sont couramment utilisés et ont été validés par de nombreuses études :
- le test de l'espace ouvert (« open field ») : Il consiste en l'exposition forcée de l'animal (rat ou souris) à un milieu nouveau et à l'observation de son comportement. L'animal est généralement placé dans une cage ou boîte dont le sol est quadrillé et qu'il ne connaît pas. L'activité locomotrice et le comportement sont alors évalués en enregistrant le nombre de franchissement des lignes du quadrillage, la fréquence des reculs, le nombre d'entrées et le temps passé dans les carrés centraux. Ce modèle animal est considéré comme un bon modèle de l'anxiété « normale » lors de l'adaptation à un nouvel environnement. Il a une bonne prédictibilité de la réponse humaine aux traitements (L. Prut, European Journal of Pharmacology 463 (2003) 3- 33). Le test de l'espace ouvert est également utile, lorsqu'il est prolongé (temps d'enregistrement du comportement sur 2 heures), car il permet de suivre l'activité motrice des animaux et donc leur état de veille, tandis que les mesures au temps courts donnent une information sur l'anxiété.
- le test de la boîte éclairée-obscure (« light-dark box ») : un autre modèle animal de l'anxiété s'appuie sur les propriétés aversives d'un espace ouvert (« open-field ») et sur la comparaison des activités exploratoires dans un compartiment illuminé et dans un compartiment sombre sous influence de substances anxiolytiques (M Bourin, European Journal of Pharmacology 463 (2003) 55-65). Ce paradigme est fondé sur l'aversion innée des rongeurs pour les lieux lumineux et sur le comportement exploratoire spontané des rongeurs en réponse à des milieux stressants (environnement nouveau ou lumière). L'appareil de test est divisé en deux compartiments, l'un est petit, sombre et sûr, le second est vaste, lumineux et stressant. Ce paradigme permet de prédire des activités de type anxiolytique ou anxiogénique des médicaments sur des souris. Les passages entre chaque compartiment sont un indice de l'activité d'exploration, tandis que le temps passé dans chaque compartiment est un reflet de l'aversion. Des anxiolytiques, efficaces chez l'être humain, classiques (benzodiazépines) aussi bien que des composés anxiolytiques plus récents (médicaments sérotoninergiques ou agissant sur les récepteurs neuropeptidiques) peuvent être détectés par ce modèle animal qui a l'avantage d'être simple et rapide d'utilisation.

Le test de l'espace ouvert avec gradient lumineux : les deux modèles décrits plus haut peuvent être judicieusement couplés afin de vérifier le potentiel anxiolytique d'une molécule, en éliminant l'effet « terrier » induit par la présence du compartiment obscur.

### Utilisation de modèles animaux pour la dépression

### - le test de la nage forcée :

Ce test est utilisé depuis de nombreuses années avec des rats ou des souris, pour tester l'activité des antidépresseurs (F. Borsini, Psychopharmacology (1988) 94:147-160). Il consiste à placer l'animal dans un bocal empli d'eau à température ambiante. Après 15 minutes (session de pré-test), l'animal est sorti de l'eau et séché. Vingt-quatre heures après, l'animal est de nouveau exposé aux conditions précédentes et la durée d'immobilité totale pendant une période de 5 minutes est enregistrée (session de test). L'animal est jugé immobile lorsqu'il n'effectue que les mouvements nécessaires pour garder la tête hors de l'eau. Cette immobilité est plus facile à évaluer chez le rat que chez la souris qui présente des mouvements moins explicites. Initialement, le fait que l'animal soit plus immobile lors de la deuxième immersion, apparaissait comme un indicateur du fait que l'animal avait appris qu'il ne pouvait pas s'échapper lors du pré-test. Cette immobilité semblait donc être une indication d'un certain « désespoir » de l'animal. Bien que cette interprétation soit désormais plus nuancée, car il est possible d'expliquer cette immobilité augmentée par d'autres raisons (réponse adaptée à une situation déjà connue...), ce test reste l'un des paradigmes phares de la dépression et donne de très bons résultats des activités antidépressives des médicaments.

### Utilisation de modèles animaux pour l'ESPT

De nombreux groupes de recherche (par exemple ceux de Pier-Vincenzo Piazza et Aline Desmedt de l'INSERM) ont montré que les difficultés de mémorisation associées à l'ESPT ne sont pas spécifiques à l'être humain et sont retrouvées chez les rongeurs (voir par exemple Kaouane N. Science 335, 1510 (2012).

Des modèles animaux sont donc régulièrement utilisés pour comprendre les mécanismes d'apparition de l'ESPT (Zovkic, Neuropsychopharmacology REVIEWS (2013) 38, 77-93) ou pour tester les traitements potentiels de l'ESPT (par exemple dans le brevet JP2007148435).

Plusieurs modèles animaux ont été développés pour mimer les événements traumatiques qui induisent des symptômes de peur intense et récurrente caractéristiques de l'ESPT.

### Conditionnement Pavlovien à la peur des animaux :

Ce modèle animal consiste en un conditionnement à la peur de type Pavlovien (Zovkic, Neuropsychopharmacology Reviews (2013) 38, 77-93) : les souris (ou rats) sont entraînés à associer l'exposition au danger (choc électrique...) à la présence d'un indice ou d'un environnement spécifique (indice sonore par exemple). Le souvenir de cette association (danger-indice) est testé plus tard en mesurant le comportement de tétanie (par la peur) lorsque l'animal est ré-exposé à l'indice de conditionnement en l'absence de danger réel.

Le conditionnement par la peur présente de nombreux avantages comme modèle d'ESPT. Tout d'abord, les rongeurs, tout comme les patients souffrant d'ESPT, sont soumis à un événement traumatisant et la mémoire de cet événement persiste sur de longues durées. Ensuite, les reviviscences de l'évènement traumatique ou les évitements typiques de l'ESPT peuvent être modélisés par l'exposition du rongeur à l'indice sonore ou au contexte associé au danger, sans que cela nécessite une ré-exposition réelle à l'évènement traumatique lui-même (choc électrique). De plus, l'hyperactivité du système nerveux observé chez les patients souffrant d'ESPT peut être évaluée en comparant l'amplitude et l'apparition des sursauts produits en réponse à un son leur rappelant le traumatisme. Enfin, les études sur l'ESPT ont montré que les régions du cerveau impliquées sont notamment l'amygdale, l'hippocampe et le cortex préfrontal qui sont aussi impliquées dans le conditionnement par la peur des animaux.

Grâce à ce modèle animal, il est donc possible de tester l'influence de différentes variables expérimentales, ou de différents traitements sur la mémoire pathologique de la peur, en observant simplement si elles augmentent, diminuent ou suppriment le comportement de tétanie. On peut ainsi augmenter ou diminuer la sévérité de l'évènement traumatique en diminuant l'intensité du choc électrique et tenter de la relier à la persistance et à la force de la mémoire de cette peur.

D'autres modèles animaux sont aussi couramment utilisés comme paradigmes de l'ESPT comme par exemple l'exposition à un ou plusieurs prédateurs, avec divers degrés de contact (exposition à l'odeur, à l'animal lui-même...).

Dans ce cas aussi, l'occurrence des symptômes de l'ESPT sont évalués après un certain délai, sans exposition au danger, pour simuler l'apparition à long terme de la pathologie de l'ESPT.

### Extinction de la peur :

Après un conditionnement à la peur tel que décrit précédemment il peut être intéressant d'appliquer un protocole d'extinction de cette peur. Classiquement, cette phase est placée 24 heures après la phase de conditionnement.

Pour cela, l'animal conditionné est entraîné à ce que l'indice de conditionnement (le son) ne prédise plus l'occurrence du danger (le choc électrique).

Bien que l'extinction puisse apparaître comme l'annulation de l'apprentissage à la peur précédent, elle n'est pas équivalente à un effacement de la mémoire ou à un oubli définitif, puisque, d'une part, la tétanie n'est pas complètement éliminée par la phase d'extinction et, d'autre part, que la réponse au stimulus peut réapparaitre spontanément ou suite à un choc. Les protocoles d'extinction modélisent le ré-apprentissage de la réponse normale (non motivée par la peur) à l'indice (sonore) et sont très utiles dans la modélisation de l'ESPT.

### Stabilisation de la peur :

Il peut être utile, pour évaluer la performance d'un traitement, d'ajouter, après la phase d'extinction une phase de stabilisation, espacée de 24h.Typiquement, cette phase place, à nouveau, les animaux dans les conditions de la phase d'extinction. Elle permet alors de détecter des performances décalées des traitements, mais aussi des résurgences de la tétanie (effet rebond).

Elle permet aussi de modéliser un ESPT « consolidé », c'est-à-dire la situation où un individu exposé à un traumatisme psychologique (ponctuel ou prolongé) et traité (ou non) par des méthodes conventionnelles, présente les troubles résiduels de ce trauma.

### Utilisation de modèles animaux pour l'apparition de troubles schizophréniques

### Test d'inhibition pré-impulsion (test "PPI")

Une des problématiques importantes des traitements ciblant le système nerveux central reste l'obtention de l'effet désiré sans l'apparition d'effets secondaires psychologiques handicapants, voire dépassant le bénéfice thérapeutique du traitement. Ces effets sont en effet souvent importants (effet rebond des benzodiazépines), voire paradoxaux (effet anxiogène des inhibiteurs de recapture de sérotonine à court terme). Ils peuvent, en particulier induire une déstabilisation du filtrage sensoriel et générer des symptômes analogues à la schizophrénie.

Le test d'inhibition pré-impulsion est fondé sur la capacité du cerveau à s'adapter à un stimulus d'intensité modérée de façon à limiter l'effet de ce même stimulus à forte intensité.

Dans ce test, les animaux sont placés dans une cage insonorisée et soumis, soit à une impulsion sonore de 120 décibel (dB), soit à une séquence de 2 impulsions espacées d'une centaine de millisecondes, la seconde impulsion étant d'intensité fixe de 120 dB et le premier d'intensité variable entre 70 et 85 dB).

Chez un animal normal, la première impulsion sonore a la capacité d'inhiber partiellement le sursaut causé par le son d'intensité maximale. Chez des animaux présentant des troubles psychotiques de nature schizophrénique, cette inhibition est plus ou moins éliminée.

Le test PPI chez le rongeur est un test puissant dans la mesure où il est reproductible chez l'homme chez qui il permet de détecter les troubles schizophréniques, ainsi que l'effet indésirable des traitements pharmaceutiques (PCP, benzodiazépine, kétamine).

Dans les exemples suivants, les effets de la memantine, du propranolol et de la fluoroéthylnormemantine (FENM, objet de la présente invention) sont étudiés chez des rats naïfs (Wistar) soumis à différents tests reliés à la dépression ou à l'anxiété, puis comparés.

Les substances testées sont administrées aux rats par voie intrapéritonéale sous un volume de 5ml/kg, 30 minutes avant d'effectuer les tests comportementaux.

Les solutions injectées contiennent l'une ou l'autre des molécules sous la forme de l'un de leur sel, un chlorhydrate (memantine.HCl et FENM.HCl) pour les dérivés azotés et tel quel pour le propranolol, aux concentrations indiquées ci-dessous dans un mélange 95% (NaCl 0.9% ww dans l'eau) / 5% Ethanol. Dans chaque expérience, la dose par kilogramme est référencée.

De cette façon, on administre aux animaux un volume constant de solution (aux différences de poids près) égal à 5ml/kg soit 1.5 ml pour un rat de 300 g.

Pour chaque paradigme, et chaque solution testée, 12 rats sont soumis à l'injection et au test de comportement. La solution de contrôle est une solution d'eau salée.

### Exemple 1 : traitement de l'anxiété et activité locomotrice

### Test de l'espace ouvert :

L'activité locomotrice des animaux, et l'anxiété « normale » liée à la découverte d'un milieu inconnu sont notamment mesurées dans un champ ouvert (« open-field ») grâce à un système de suivi vidéo pendant 2 heures et ce, 30 minutes après l'injection des solutions à tester. Les données enregistrées sont notamment, avec un relevé toutes les 5 minutes, le temps passé dans les carrés centraux, l'activité verticale, et le nombre de déplacements.

Les différents tableaux 1 à 3, ci-dessous, rapportent les effets de la FENM.HCl, en comparatif de la memantine et d'une solution saline de contrôle.

**TABLEAU 1**

| **Groupe d'animaux** | **Temps moyen passé dans les carrés centraux par intervalle de temps (s)** | | | |
|---|---|---|---|---|
| | 0-5 min | 5-10 min | 0- 10 min | 0-120 min |
| Control | 132,9 | 104,9 | 237,8 | 2293,4 |
| Mémantine 10 mg/kg | 90,4 | 46,3 | 136,8 | 1874,8 |
| FENM 5 mg/kg | 128,8 | 88,0 | 216,8 | 1563,6 |
| FENM 10 mg/kg | 116,8 | 109,9 | 226,7 | 1803,8 |
| FENM 20 mg/kg | 129,8 | 105,9 | 235,7 | 1692,8 |

**TABLEAU 2**

| **Groupe d'animaux** | **Activité verticale** | | | |
|---|---|---|---|---|
| | 0 - 5 min | 5 - 10 min | 0 - 10 min | 0 - 120 min |
| Control | 49,83 | 26,58 | 76,42 | 115,33 |
| Mémantine 10 mg/kg | 11,33 | 4,67 | 16,00 | 60,08 |
| FENM 5 mg/kg | 40,67 | 30,50 | 71,17 | 136,00 |
| FENM 10 mg/kg | 52,33 | 34,08 | 86,42 | 173,75 |
| FENM 20 mg/kg | 47,33 | 19,25 | 66,58 | 208,92 |

**TABLEAU 3**

| **Groupe d'animaux** | **Nombre de déplacements** |
|---|---|
| | 0 - 120 min |
| Control | 1650 |
| Mémantine 10 mg/kg | 750 |
| FENM 5 mg/kg | 1600 |
| FENM 10 mg/kg | 1800 |
| FENM 20 mg/kg | 2100 |

Les résultats des différentes mesures montrent que la FENM.HCl, même aux doses élevées, ne génère pas l'effet de somnolence observé pour la memantine qui diminue le niveau d'activité global des animaux, sans améliorer le temps de présence au centre du champ. Cet effet est particulièrement visible sur le temps long de 2 heures de l'expérience.

### Exemple 2 : traitement de l'anxiété (paradigme de la boîte éclairée-obscure)

Dans cet exemple le test de la boîte éclairée-obscure a été utilisé. Comme il est basé sur l'aversion innée des rongeurs pour la lumière, l'augmentation du temps passé dans le compartiment éclairé est donc considérée comme rendant compte d'un niveau d'anxiété plus faible.

La boîte utilisée pour ce test est divisée en un comportement blanc éclairé à forte puissance (600 lux) et un comportement noir possédant un couvercle (environ 5 lux). Le nombre d'entrées et le temps passé dans le compartiment éclairé (zone anxiogène) sont enregistrés pendant 10 minutes par un système de suivi vidéo. Le système permet en outre de différencier les périodes entre 0 et 5 minutes et entre 5 et 10 minutes.

Le tableau 4 ci-dessous montre les temps de présence dans le compartiment éclairé pour différentes doses de FENM.HCl, en comparatif de la memantine et d'une solution saline de contrôle

**TABLEAU 4**

| **Groupe d'animaux** | **Temps passé dans le compartiment éclairé (s)** | | |
|---|---|---|---|
| | **0 - 5 min** | **5 - 10 min** | **0 - 10 min** |
| Control | 34,1 | 37,0 | 71,1 |
| Mémantine 1 mg/kg | 34,2 | 25,7 | 59,9 |
| Mémantine 3 mg/kg | 26,2 | 8,1 | 34,3 |
| Mémantine 10 mg/kg | 31,0 | 0,9 | 32,0 |
| FENM 1 mg/kg | 42,4 | 28,6 | 71,0 |
| FENM 3 mg/kg | 34,4 | 33,2 | 67,5 |
| FENM 10 mg/kg | 96,8 | 75,0 | 171,9 |

Les résultats montrent une augmentation importante et significative (test t de Student p<0,01) du temps passé dans le compartiment éclairé de la boîte pour les animaux traités avec la solution FENM 10mg/kg, comparativement au control, alors qu'aucun effet n'est observé pour la memantine. Ce résultat démontre l'effet anxiolytique du composé selon l'invention et ainsi qu'un mode d'action et un effet différent par rapport à la memantine.

Cela démontre bien que la mémantine n'a aucun effet anxiolytique.

### Exemple 3 : traitement de l'anxiété (paradigme du champ éclairé par un gradient lumineux)

Dans cet exemple le test de la boîte éclairée-obscure a été modifié, par élimination du compartimentage de la boite et utilisation d'un gradient lumineux de forte intensité (600 lux). Comme il est basé sur l'aversion innée des rongeurs pour la lumière, l'augmentation du temps passé dans la zone la plus proche de la source lumineuse est donc considérée comme rendant compte d'un niveau d'anxiété plus faible.

Les animaux sont exposés à une séquence de 12 minutes, décomposée en trois parties, 4 minutes dans l'obscurité (luminosité ambiante de 5 lux), 4 minutes en présence du gradient lumineux et 4 minutes de nouveau dans l'obscurité. Les déplacements sont enregistrés de la même façon que pour le test de champ ouvert. Le champ d'observation est divisé en 4 zones numérotées de 1 à 4 en fonction de l'éloignement croissant par rapport à la source lumineuse.

Le tableau 5 ci-dessous montre les effets de différentes doses de FENM.HCl, comparativement à la solution de contrôle.

**TABLEAU 5**

| **Groupe d'animaux** | **Pourcentage du temps passé dans la zone la plus proche de la source lumineuse** | | |
|---|---|---|---|
| | Obscurité 1 (Habituation) | Lumière 600 lux (Stress) | Obscurité 2 (Retour au calme) |
| Control | 32% | 15% | 30% |
| FENM 5 mg/kg | 32% | 22% | 30% |
| FENM 10 mg/kg | 29% | 30% | 34% |
| FENM 20 mg/kg | 39% | 21% | 29% |

Les résultats montrent, pour les animaux traités par la FENM.HCl, une réduction significative de l'évitement par les animaux de la zone la plus fortement éclairée, signifiant, une diminution nette de l'anxiété. Dans le cas de la dose de 10mg/kg, l'effet d'anxiété est complètement éliminé et les animaux passent autant de temps dans la zone proche de la source, en présence et en absence de lumière.

Par ailleurs, cet effet d'annulation ou de réduction de l'anxiété est constaté sans autre effet notoire sur le comportement des animaux et, en particulier, sur leur capacité à se déplacer.

### Exemple 4 : traitement de la dépression (nage forcée)

Au cours du test de la nage forcée, les rats (12 par groupes) sont forcés à nager dans une cuve remplie d'eau à 23°C (10 minutes le premier jour pour permettre l'accoutumance au milieu et 6 minutes le deuxième jour).

Le comportement des rats (arrêt de la nage, baisse d'activité) est quantifié par un système de suivi vidéo.

Les solutions thérapeutiques à tester sont administrées le deuxième jour 30 minutes avant l'expérience. Les animaux dits résignés sont ceux qui vont présenter a) un temps d'immobilité plus important et b) passer moins de temps à tenter énergiquement (frénétiquement) de sortir du bécher, soit donc un comportement de type « dépressif ». Le tableau 6 ci-dessous rapporte les effets constatés sur ces deux paramètres en présence de différentes doses de FENM.HCl, en comparatif de la memantine et d'une solution saline de contrôle.

**TABLEAU 6**

| **Groupe d'animaux** | **Comportement des rats sur la période de temps 1 à 6 min** | |
|---|---|---|
| | **immobilité (s)** | **mouvements frénétiques (s)** |
| Control | 177,5 | 35,6 |
| Mémantine 1 mg/kg | 139,8 | 55,1 |
| Mémantine 3 mg/kg | 145,7 | 48,7 |
| Mémantine 10 mg/kg | 119,7 | 67,2 |
| FENM 1 mg/kg | 157,8 | 43,2 |
| FENM 3 mg/kg | 164,3 | 47,6 |
| FENM 10 mg/kg | 140,5 | 60,6 |

Les résultats montrent une décroissance très significative (p<0,001) de la durée d'immobilité et une augmentation significative des effets de frénésie, démontrant ainsi des effets antidépresseurs comparables pour la memantine et le composé selon l'invention (FENM). Cet effet est observé quelle que soit la dose et est particulièrement notable à la dose 10mg/kg.

### Exemple 5 : traitement de l'ESPT (Conditionnement à la peur + extinction + stabilisation)

Les protocoles d'extinction modélisant le ré-apprentissage de la réponse normale (non motivée par la peur) à l'indice (sonore) sont très utiles dans la modélisation de l'ESPT.

La demanderesse a utilisé un test de conditionnement à la peur dans lequel l'indice (stimulus conditionnel neutre) est un son, couplé à un danger (stimulus inconditionnel aversif) qui est un choc électrique délivré par le plancher de la boîte. Ce conditionnement est suivi par une phase d'extinction de la peur avec injection des solutions de traitement à tester puis par une phase de test proprement dite.

Phase de conditionnement à la peur (FC) : le conditionnement par la peur a lieu dans une boîte de conditionnement standard (contexte A, boite blanche dans le Tableau 7) et les rats (8 par groupe) sont soumis à 3 couples « son-électrochoc » à des temps de 180s, 381s et 582s après leur placement dans la boîte. Chaque son dure 20 s et chaque électrochoc dure 1s avec une intensité de 1mA.

La phase d'extinction de la peur (Ext.) se déroule de la manière suivante : les rats sont placés dans une boîte semblable à celle qui sera utilisée pour le test final (contexte B, boîte rose dans le Tableau 7), pendant une session de 25 minutes. Lors de cette session, ont lieu 24 présentations du même son que celui utilisé pendant la session de conditionnement (durée de 20s chacun, séparés de 35 s). Bien sûr, aucun choc électrique n'est délivré, on enregistre le pourcentage du temps de tétanie (« freezing ») pendant la phase.

Phase de stabilisation : le jour suivant la phase d'extinction, 30 minutes avant la phase de test, les rats reçoivent une injection d'eau salée, puis on teste leur réponse à la peur dans le contexte B en les soumettant à une séquence de 3 sons (même séquence que celle utilisée pour le conditionnement), en enregistrant le pourcentage de temps d'immobilité pendant cette phase de test.

Le tableau 8 ci-dessous rapporte les effets constatés sur le pourcentage de temps passé immobile par les animaux, durant la phase de stabilisation, pour différentes doses de FENM.HCl, en comparatif du propranolol et d'une solution saline de contrôle, dans trois conditions de traitement :
- Effet protecteur - soit une injection 30 minutes avant la phase de conditionnement
- Traitement précoce - soit une injection 30 minutes avant la phase d'extinction
- Traitement tardif - soit une injection 30 minutes avant la phase de stabilisation

**TABLEAU 8**

| **Groupe d'animaux** | **Pourcentage du temps de tétanie (freezing) pendant la phase de stabilisation** | | |
|---|---|---|---|
| | Effet protecteur | Traitement précoce | Traitement tardif |
| Control | 37% | 33% | 28% |
| Propranolol 10mg/kg | 28% | 26% | 29% |
| FENM 5mg/kg | 23% | 21% | 26% |
| FENM 10mg/kg | 6% | 25% | 4% |
| FENM 20mg/kg | 2% | 6% | 2% |

Ces résultats montrent que la FENM a le potentiel d'éliminer quasi complètement (p<0,001) le phénomène de tétanie au stade de la phase de stabilisation, aussi bien lorsqu'elle est administrée au moment du conditionnement que lors du traitement post traumatique, précoce ou tardif. Le propranolol ne se différencie pas du témoin au niveau de la phase de stabilisation alors qu'il améliore le phénomène d'extinction, ce qui est conforme avec les données publiées de la littérature sur le rebond après sevrage.

Par ailleurs, on notera également qu'un effet rebond significatif (augmentation de la tétanie) est observé (+25% par rapport au control) lors de la phase de stabilisation lorsque la memantine est administrée à 10 mg/kg lors de la phase d'extinction.

### Exemple 6 : absence de perturbation du « filtrage sensoriel » (test PPI).

Le test d'inhibition pré-impulsion est fondé sur la capacité du cerveau à s'adapter à un stimulus d'intensité modérée de façon à limiter l'effet de ce même stimulus à forte intensité. Dans ce test, les animaux (12 par groupe), sont placés dans une cage insonorisée et soumis, soit à un impulsion sonore de 120 décibel (dB), soit à une séquence de 2 impulsions espacées d'une centaine de millisecondes, la seconde impulsion étant d'intensité fixe de 120 dB et la première d'intensité variable entre 70 et 85 dB).

Chez un animal normal, la première impulsion sonore a la capacité d'inhiber partiellement le sursaut causé par le son d'intensité maximale. Chez des animaux présentant des troubles psychotiques de nature schizophrénique, cette inhibition est plus ou moins éliminée.

Le tableau 9 ci-dessous rapporte les effets constatés sur ces deux paramètres en présence de différentes doses de FENM.HCl, par comparaison avec la Mémantine ou avec une solution saline de contrôle.

**TABLEAU 9**

| **Groupe d'animaux** | **Pourcentage d'inhibition pré-pulse en fonction de l'intensité du premier pulse** | | | | |
|---|---|---|---|---|---|
| | 72 dB | 76 dB | 80 dB | 84 dB | Moyenne |
| Control | 38% | 46% | 54% | 66% | 51% |
| Mémantine 10 mg/kg | 19% | 24% | 36% | 44% | 31% |
| FENM 5mg/kg | 24% | 37% | 47% | 60% | 42% |
| FENM 10 mg/kg | 27% | 39% | 51% | 64% | 45% |
| FENM 20mg/kg | 31% | 46% | 54% | 62% | 48% |

Ces résultats montrent que, alors que la Mémantine à 10mg/kg, induit une inhibition de l'effet PPI (p<0,01, effet comparable aux effets reportés dans la littérature par ailleurs pour ce composé), la FENM, n'induit aucun effet, même à forte dose (20mg/kg). Cela signifie que la mémantine présente l'effet indésirable d'engendrer de la schizophrénie, tandis que la FENM ne présente pas l'effet indésirable d'engendrer de la schizophrénie.

Dans ce test, la performance de la Mémantine, et, en creux, l'absence de performance de la FENM, démontre un effet secondaire schyzophréniant de cette Memantine. Cet effet est particulièrement indésirable. En effet il est indésirable car non seulement ce type de comportement est source de souffrance pour le patient mais, qui plus est, il génère un stress anxiogène qui va augmenter l'anxiété au contraire de la diminuer.

## Revendications

1. Composé de formule (I) ou un de ses sels pharmaceutiquement acceptable 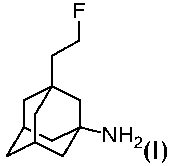 pour son utilisation pour le traitement ou la prévention d'un trouble anxieux chez un patient.

2. Composé pour son utilisation selon la revendication 1, **caractérisé en ce que** le sel pharmaceutiquement acceptable correspond à la formule (II) 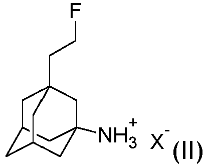 dans laquelle, X⁻ désigne un contre anion choisi dans le groupe constitué par les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate et tosylate.

3. Composé pour son utilisation selon l'une des revendications 1 ou 2, **caractérisé en ce que** le trouble anxieux est associé à une dépression.

4. Composé pour son utilisation selon l'une des revendications 1 ou 2, **caractérisé en ce que** le trouble anxieux observé est associé à des insomnies.

5. Composé pour son utilisation selon l'une des revendications 1 à 4, **caractérisé en ce que** le trouble anxieux est choisi dans le groupe constitué par l'état de stress post-traumatique (ESPT), la phobie spécifique, la phobie sociale, le trouble d'anxiété généralisée, le trouble panique avec ou sans agoraphobie et le trouble obsessionnel compulsif.

6. Composé pour son utilisation selon l'une des revendications 1 à 5, **caractérisé en ce qu'**il est utilisé pour un traitement de prévention de l'apparition des troubles anxieux liés à un stress post traumatique.

7. Composé pour son utilisation selon l'une des revendications 1 à 5, **caractérisé en ce que** le trouble anxieux est lié à un stress post traumatique, en particulier un stress post traumatique installé.

8. Composé pour son utilisation selon l'une des revendications 1 à 7, **caractérisé en ce que** le trouble anxieux est accompagné de crises et d'épisodes d'angoisse aigus.

9. Produit de combinaison, en particulier pour une utilisation simultanée, séparée ou étalée dans le temps, comprenant les composants suivants : un composant (A) comprenant un composé de formule (I) ou un de ses sels pharmaceutiquement acceptable et au moins un excipient pharmaceutiquement acceptable 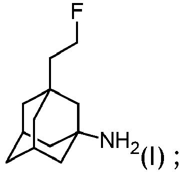 un composant (B) comprenant du propranolol et au moins un excipient pharmaceutiquement acceptable.

10. Produit de combinaison comprenant les composants suivants : un composant (A) comprenant un composé de formule (I) ou un de ses sels pharmaceutiquement acceptable 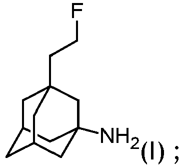 un composant (C) comprenant un antidépresseur ; dans lequel chacun des composants (A) et (C) est formulé avec au moins un excipient pharmaceutiquement acceptable.

11. Produit selon la revendication 9, comprenant en outre en combinaison, un composant (D) comprenant de la ketamine formulé avec un excipient pharmaceutiquement acceptable.

12. produit selon la revendication 10, comprenant en outre en combinaison, un composant (D) comprenant de la ketamine formulé avec un excipient pharmaceutiquement acceptable.

13. Produit selon la revendication 10, dans lequel les composants (A) et (C) sont adaptés pour une administration simultanée, séparée ou étalée dans le temps.

14. Produit selon l'une des revendications 9 à 13, pour son utilisation dans la prévention ou le traitement d'un trouble anxieux chez un patient.

15. Produit selon la revendication 9, pour son utilisation dans la prévention ou le traitement d'un trouble anxieux chez un patient, ledit patient ayant subi des abus répétés, en particulier durant l'enfance.

## Patentansprüche

1. Verbindung der Formel (I) oder ein pharmazeutisch annehmbares Salz davon 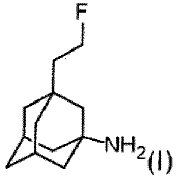 für ihre Verwendung bei der Behandlung oder Prävention einer Angststörung bei einem Patienten.

2. Verbindung für ihre Verwendung nach Anspruch 1, **dadurch gekennzeichnet, dass** das pharmazeutisch annehmbare Salz der Formel (II) 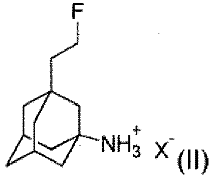 entspricht, wobei X⁻ ein Gegenanion bezeichnet, das aus der Gruppe ausgewählt ist, die zusammengesetzt ist aus Chlorid, Bromid, Iodid, Acetat, Methansulfonat, Benzolsulfonat, Camphosulfonat, Tartrat, Dibenzoat, Ascorbat, Fumarat, Citrat, Phosphat, Salicylat, Oxalat, Bromohydrat und Tosylat.

3. Verbindung für ihre Verwendung nach einem der Ansprüche 1 oder 2, **dadurch gekennzeichnet, dass** die Angststörung mit einer Depression verbunden ist.

4. Verbindung für ihre Verwendung nach einem der Ansprüche 1 oder 2, **dadurch gekennzeichnet, dass** die beobachtete Angststörung mit Schlaflosigkeit verbunden ist.

5. Verbindung für ihre Verwendung nach einem der Ansprüche 1 bis 4, **dadurch gekennzeichnet, dass** die Angststörung aus der Gruppe ausgewählt ist, die zusammengesetzt ist aus posttraumatischer Belastungsstörung (PTBS), spezifischer Phobie, sozialer Phobie, generalisierter Angststörung, Panikstörung mit oder ohne Agoraphobie und Zwangsstörung.

6. Verbindung für ihre Verwendung nach einem der Ansprüche 1 bis 5, **dadurch gekennzeichnet, dass** sie für eine Behandlung zur Verhinderung des Auftretens von Angststörungen, die mit posttraumatischem Stress verbunden sind, verwendet wird.

7. Verbindung für ihre Verwendung nach einem der Ansprüche 1 bis 5, **dadurch gekennzeichnet, dass** die Angststörung mit posttraumatischem Stress, insbesondere mit installiertem posttraumatischem Stress, verbunden ist.

8. Verbindung für ihre Verwendung nach einem der Ansprüche 1 bis 7, **dadurch gekennzeichnet, dass** die Angststörung von akuten Angstanfällen und -episoden begleitet wird.

9. Kombinationsprodukt, insbesondere zur gleichzeitigen, getrennten oder zeitlich verteilten Anwendung, umfassend die folgenden Komponenten: Eine Komponente (A), die eine Verbindung der Formel (I) oder ein pharmazeutisch annehmbares Salz davon und mindestens einen 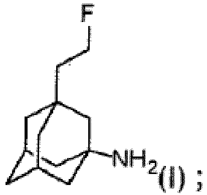 pharmazeutisch annehmbaren Hilfsstoff umfasst
eine Komponente (B), die Propranolol und mindestens einen pharmazeutisch annehmbaren Hilfsstoff umfasst.

10. Kombinationsprodukt, umfassend die folgenden Komponenten: Eine Komponente (A), umfassend eine Verbindung der Formel (I) oder ein pharmazeutisch annehmbares Salz davon 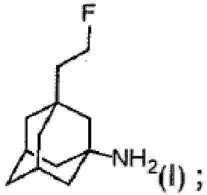 eine Komponente (C), umfassend ein Antidepressivum; wobei jede der Komponenten (A) und (C) mit mindestens einem pharmazeutisch annehmbaren Hilfsstoff formuliert ist.

11. Produkt nach Anspruch 9, weiter umfassend in Kombination eine Komponente (D), die Ketamin umfasst, formuliert mit einem pharmazeutisch annehmbaren Hilfsstoff.

12. Produkt nach Anspruch 10, weiter umfassend in Kombination eine Komponente (D), die Ketamin umfasst, formuliert mit einem pharmazeutisch annehmbaren Hilfsstoff.

13. Produkt nach Anspruch 10, wobei die Komponenten (A) und (C) für eine gleichzeitige, getrennte oder zeitlich gestaffelte Verabreichung geeignet sind.

14. Produkt nach einem der Ansprüche 9 bis 13 für seine Verwendung bei der Prävention oder Behandlung einer Angststörung bei einem Patienten.

15. Produkt nach Anspruch 9 für seine Verwendung bei der Prävention oder Behandlung einer Angststörung bei einem Patienten, wobei der Patient wiederholt, insbesondere in der Kindheit, missbraucht wurde.

## Claims

1. A compound of formula (I) or one of the pharmaceutically acceptable salts thereof 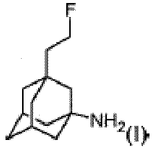 for use for treating or preventing of an anxiety disorder in a patient.

2. The compound for the use thereof according to claim 1, **characterised in that** the pharmaceutically acceptable salt corresponds to the formula (II) 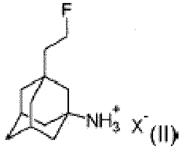 wherein X⁻ designates a counter anion selected from the group consisting of chloride, bromide, iodide, acetate, methane sulfonate, benzene sulfonate, camphorsulfonate, tartrate, dibenzoate, ascorbate, fumarate, citrate, phosphate, salicylate, oxalate, hydrobromide and tosylate ions.

3. The compound for the use thereof according to one of claims 1 or 2, **characterised in that** the anxiety disorder is associated with depression.

4. The compound for the use thereof according to one of claims 1 or 2, **characterised in that** the observed anxiety disorder is associated with insomnia.

5. The compound for the use thereof according to one of claims 1 to 4, **characterised in that** the anxiety disorder is selected from the group consisting of post-traumatic stress disorder (PTSD), specific phobia, social phobia, generalized anxiety disorder, panic disorder with or without agoraphobia and obsessive-compulsive disorder.

6. The compound for the use thereof according to one of claims 1 to 5, **characterised in that** it is used for a treatment for preventing the onset of anxiety disorders linked to post-traumatic stress.

7. The compound for the use thereof according to one of claims 1 to 5, **characterised in that** the anxiety disorder is related to post-traumatic stress, in particular to established post-traumatic stress.

8. The compound for the use thereof according to one of claims 1 to 7, **characterised in that** the anxiety disorder is accompanied by crises and acute anguish episodes.

9. A combination product, in particular for a use which is simultaneous, separate or spread over time, comprising the following components: a component (A) comprising a compound of formula (I) or one of the pharmaceutically acceptable salts thereof and at least one pharmaceutically acceptable excipient 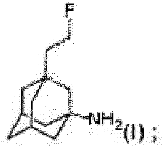 a component (B) comprising propranolol and at least one pharmaceutically acceptable excipient.

10. The combination product comprising the following components: a component (A) comprising a compound of formula (I) or one of the pharmaceutically acceptable salts thereof 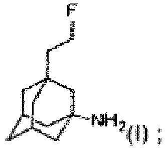 a component (C) comprising an antidepressant; wherein each of the components (A) and (C) is formulated with at least one pharmaceutically acceptable excipient.

11. A product according to claim 9, further comprising in combination, a component (D) comprising ketamine formulated with a pharmaceutically acceptable excipient.

12. The product according to claim 10, further comprising in combination, a component (D) comprising ketamine formulated with a pharmaceutically acceptable excipient.

13. The product according to claim 10, wherein the components (A) and (C) are adapted for an administration which is simultaneous, separate or spread over time.

14. The product according to one of claims 9 to 13, for the use thereof in the prevention or treatment of an anxiety disorder in a patient.

15. The product according to claim 9, for the use thereof in the prevention or treatment of an anxiety disorder in a patient, said patient having undergone repeated abuse, in particular during childhood.
